# EUROPEAN PATENT APPLICATION

(11) **EP 3 871 700 A1**
(43) Date of publication of application: **01.09.2021**
(21) Application number: 20159565.9
(22) Date of filing: 26.02.2020
(51) Int. Cl.: A61L 2/28

(54) **DISINFECTION SYSTEM MEASURING TEMPERATURE**

(71) Applicant: Fresenius Medical Care Deutschland GmbH, 61352 Bad Homburg (DE)
(72) Inventor: Kopperschmidt, Pascal, 97456 Dittelbrunn (DE)
(74) Representative: Bobbert & Partner Patentanwälte PartmbB

(57) **Abstract**

The present invention relates to a disinfection system (100) for use by a user when disinfecting the skin of a user. The disinfection system (100) comprising a reservoir (101) for a disinfection fluid (107) or a receptacle (103) for a reservoir (101) for a disinfection fluid (107). It further comprises an applicator (105) for dispensing disinfection fluid (107) from the reservoir (101) to the user upon request. A temperature measuring device (120) for measuring temperature values (200, 201) on or above a body part of the user, particularly of a skin section is also comprised by the disinfection system (100). Moreover, the disinfection system (100) comprises an evaluation device (130) for evaluating temperature values (200, 201) measured by the temperature measuring device (120). An output device (140) configured to emit a signal for the attention of the user is also comprised by the disinfection system (100), wherein the signal is based on an evaluation carried out by the evaluation device (130) of at least one first temperature value (200) measured by the temperature measuring device (120).

## Description

The present invention relates to a disinfection system according to the preamble of claim 1. It also relates to a method according to the preamble of claim 13.

From practice automatic disinfection dispensers comprising an applicator for applying disinfectant are known.

It is an object of the present invention to propose a further disinfection system for the disinfection of parts of a human body.

The object of the present invention is achieved by the disinfection system having the features of claim 1. It may also be achieved by the method having the features of claim 13.

According to the present invention a disinfection system is thus provided to be used when disinfecting the skin covering a body part of a patient, e.g. of an arm before the puncturing or cannulation of a blood vessel, e. g., a shunt used for performing extracorporeal blood treatment. It can be also used by a user when disinfecting the hands.

The disinfection system comprises a reservoir for a disinfection fluid. The reservoir may comprise the disinfection fluid, or it may not in case the reservoir has yet to be filled with said disinfection fluid. Instead of the reservoir, or in addition to it, the disinfection system may comprise a receptacle for a reservoir for a disinfection fluid.

Such a receptacle, if provided, may be a support for the reservoir, a holder, or the like. It may be designed to receive different types of reservoirs, or it may be prepared in order to be compatible to them.

Further, the disinfection system comprises an applicator for dispensing disinfection fluid from the reservoir (once filled) to the user upon request. The request can be made or triggered manually (e.g. by pressing the reservoir walls), by a switch, a knob, a lever, by different kinds of sensors, or the like. The applicator may be prepared and/or configured to dose and/or to release the disinfection fluid, e.g. by atomizing it via a nozzle.

Moreover, a temperature measuring device for measuring temperature values on or above a body part of the user, the part particularly being a skin section of the user, is comprised by the disinfection system according to the present invention.

Also, an evaluation device being in signal communication with the temperature measuring device is part of the disinfection system. The evaluation device is configured to serve for evaluating the temperature values measured by the temperature measuring device.

The disinfection system optionally also comprises an output device configured to emit a signal to a medical device and/or for the attention of the user. The signal may be based on the evaluation, carried out by the evaluation device, of at least a first temperature value measured by the temperature measuring device. For example, the signal may indicate that the disinfection process, for which the user has used the disinfection system has been completed (or has not (yet) been completed).

The method for disinfecting a part of the body of a patient or a user according to the present invention encompasses providing a reservoir, which comprises a disinfection fluid. Moreover, activating an applicator for dispensing disinfection fluid from the reservoir to a skin section upon request is also encompassed as a further step. Measuring at least one temperature value as a first temperature value, or several temperature values, on or above the skin section using a temperature measuring device after the disinfection fluid was dispensed is also encompassed by the method according to the present invention. As a further step, evaluating based on the at least one first temperature value measured by the temperature measuring device may also be encompassed. The evaluation may take place based on a pre-determined or pre-set reference temperature value or with a pre-determined or pre-set reference temperature range.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" as encompassing "at least one". This understanding is also equally encompassed by the present invention as the interpretation that a numerical word, for example, "one" may alternatively mean "exactly one", wherever this is evidently technically possible in the view of the person skilled in the art. Both of these understandings are encompassed by the present invention and apply herein to all used numerical words.

Advantageous developments of the present invention are each subject-matter of the dependent claims and embodiments.

Whenever an embodiment is mentioned herein, it represents an exemplary embodiment according to the present invention which is not to be understood as limiting.

Embodiments according to the present invention may comprise one or several of the features mentioned supra and/or in the following in any combination unless the person skilled in the art considers the particular combination to be technically impossible.

In some embodiments of the disinfection system, the medical device configured for receiving the signal emitted by the output device is a medical treatment device such as a blood treatment apparatus (e. g., a hemodialysis apparatus, hemofiltration apparatus or hemodiafiltration apparatus, in particular an apparatus for the acute, the chronic or the continuous renal replacement therapy (CRRT)) and/or another medical device such as a cannulation robot or machine for the automated affixing of a cannula to a patient. Such a robot is disclosed in DE 10 2017 201 434 A1, US 2019/0374700 A1, EP 0 654 244 B1, US 2015/0065916 A1 or WO 2015/052719 A1, the respective disclosures of which documents are incorporated into this specification by way of reference. The receiving medical device, or its controller, may be programmed to not allow some or all of its functions in case the received signal emitted by the output signal does not comply with pre-set conditions. For example, if the disinfection is not considered complete, or if no signal is received at all, then the medical device may - due to its programming - not allow, for example, the patient's vessel to be punctured, or the patient to be treated.

In several embodiments, the output device is programmed to emit the signal to a cloud, a network, a network of computer implemented computer programs (e.g., a client/server-system, a cloud computing system or the like.

In some embodiments of the disinfection system, the medical device forms part of it.

In some embodiments of the disinfection system, the evaluation device is programmed and/or configured to compare a temperature value taken after the disinfection fluid was dispensed, herein called the "first temperature value", with a pre-determined or pre-set reference temperature value or with a pre-determined or pre-set reference temperature range. This evaluating step may encompass establishing a mathematical relation between the first temperature value and the pre-determined or pre-set temperature value and/or between the first temperature value and the pre-determined or pre-set temperature range (e.g., "higher than", "lower than", "within" and so on). The evaluation device may be programmed to check whether any measured temperature value meets at least one pre-determined or pre-set criterion, such as a complete evaporation of the disinfectant, which guarantees a successful disinfection of the skin.

Such a criterion may be, for example, whether a measured (first) temperature value lies on or above a pre-determined or pre-set reference temperature value. Another example for a criterion may be, whether a measured temperature value lies within a pre-determined or pre-set reference temperature range.

Additionally, a further criterion may be whether a pre-determined or pre-set period of time, e.g. two minutes, three minutes etc., has passed since the dispensing of the disinfection fluid was carried out by the disinfection system.

In several embodiments, the temperature measuring device of the disinfection system is furthermore programmed to also measure a second temperature value on or above a body part of the user. Preferably, the second temperature value is measured before the disinfection fluid is dispensed to the user by the applicator, while the first temperature value is measured after the disinfection fluid was dispensed to the user by the applicator. The measuring of the second temperature value may be started manually or automatically. For example, the disinfection system may be programmed to, when disinfection is requested or triggered by the user or the patient, first measure the second temperature value and to only dispense the disinfectant after the second temperature value has been measured.

In some embodiments, the evaluating device is programmed and/or configured to use or to determine the second temperature value as the pre-determined reference temperature. Hence, the first temperature value may be compared with the second temperature value. In some embodiments, a signal, such as the signal indicating that the disinfection process has been completed, is only emitted if the comparison between second and first temperature value fulfils a predetermined criterion. For example, the criterion, to be fulfilled, may demand that the first temperature value has reached at least a pre-determined fraction of the second temperature value (e. g., 80 % of the second temperature), or has to be identical with the second temperature, or, due to heat resulting from the evaporation, exceeds the second temperature value.

In several embodiments, the evaluating device is programmed and/or configured to use the second (or first) temperature to determine or set the pre-determined reference temperature range on the basis of the second temperature value, for example by calculating a range encompassing a span below the second temperature value and the same (or another) span above the second temperature value, i.e., for example, reaching from (second temperature value - 1°C) to (second temperature value + 1°C).

It may also be encompassed by the present invention to store several, preferably subsequent, second temperature values in the disinfection system. In some embodiments, these values may be processed in order to calculate a reference temperature value, e.g. by calculating the arithmetic mean or an "average" second temperature value. In several embodiments, a reference temperature range may also be calculated on the basis of the "average" second temperature value using the calculating steps mentioned herein analogously.

In some embodiments, a signal emitted from the output device is an alarm signal, which is emitted when or if the evaluation via the evaluation device indicates that the first temperature value measured by the temperature measuring device does not meet at least one pre-determined or pre-set criterion, e.g. as mentioned herein.

The signal may be an acoustic signal, e.g. an alarm tone, and/or an optical signal, e.g. a light, e.g. from a LED, or the like, preferably in a warning color, e.g. red or orange.

In several embodiments, the signal emitted from the output device is a signal indicating that the disinfection process has been completed or is considered to be complete. For example, such a signal may be emitted when or if the evaluation via the evaluation device indicates that the first temperature value measured by the temperature measuring device does meet at least one pre-determined criterion, e.g. (at least) one of the criteria mentioned herein.

The signal may be an acoustic signal, e. g., a signal tone, and/or an optical signal, e. g., a light, e. g., from a LED or the like, preferably in a color creating positive associations, e. g., green or blue.

In several embodiments, the disinfection system may be programmed to emit either the signal indicating that the disinfection process has been completed or is considered to be complete and the signal indicating that the disinfection process has not been completed yet. For example, a green light may be provided to indicate that the disinfection process has been completed, whereas a red light may be provided signaling that the skin is not considered to be fully disinfected yet.

In some embodiments, the temperature measuring device comprises a temperature sensor or an infra-red measuring unit. In other embodiments, the measuring device is or comprises a thermometer, a resistance temperature measuring device. In some embodiments, the temperature measuring device is a pyrometer.

In several embodiments, the disinfection fluid is a disinfection solution, in particular Freska-SEPT 80 by Fresenius Kabi, Deutschland, Myxal-SEPT 80 by Peter Greven Physioderm, Deutschland, or Freka-DERM by Dr. Schuhmacher GmbH, or any other disinfection solution, preferably containing chlorhexidin.

In some embodiments, the reservoir is or comprises a bottle, or has or is in the form of a bottle.

The bottle may be made of glass and/or plastic or any other material suitable for containing disinfection fluids. Its walls may be elastic or rigid.

In several embodiments, the receptacle is or comprises a reservoir for or with a disinfection fluid.

The features mentioned above for the bottle may also apply to the reservoir without restriction.

In some embodiments, the reservoir comprises a disinfection fluid.

In several embodiments, the disinfection system comprises a controller. The controller is configured and/or programmed to control the applicator, the evaluation device, the temperature measuring device as well as the output device.

In some embodiments, the disinfection system comprises a wall mount.

The wall mount may encompass clamps or screws or the like. It is preferably designed to provide a releasable mounting, e.g. in order to allow the disinfection system to be easily cleaned.

In several embodiments, the evaluation device may also be configured and/or programmed to process the temperature values received from the temperature measuring device. This may enable the evaluation device to check the relation between the temperature values measured by the temperature measuring device and the pre-determined temperature value or the pre-determined temperature value range 203, respectively. Alternatively, or additionally, it may also check whether a pre-determined (or pre-set) criterion is fulfilled or not.

In these embodiments, the evaluation device may transmit values, e.g. representing "alarm" or "execution", respectively, directly to the output device in order to trigger the output of the corresponding signal via the output device.

In some embodiments, the disinfection system comprises neither a clock nor a timer.

In several embodiments, the output device may be or comprise a mobile device, for example a handheld device or smartphone, which is in wireless signal communication with at least the controller or the evaluation device of the disinfection system.

In some embodiments, the evaluating step of the method according to the present invention is or encompasses comparing the measured temperature value(s) with a pre-determined or pre-set reference temperature value or range, e. g. comparing the first temperature value with the second temperature value.

The reference temperature may be determined by the temperature measuring device before or just before the disinfection fluid is dispensed to the skin.

In several embodiments, for carrying out the method according to the present invention, a disinfection system according to present invention is used.

All statements made for the system or its devices also apply to the method and vice versa. In particular, methods steps corresponding to the functions disclosed herein with respect to the disinfection system or any of its components are also disclosed herewith as optional steps of the method according to the present invention. This applies, e. g., in particular to measuring the temperature as discussed with respect to the temperature measuring device disclosed, to evaluating the measured temperature(s), for outputting a signal, and for controlling the medical device.

Some or all of the embodiments according to the present invention may comprise one, several or all of the advantages mentioned above and/or in the following.

By using the disinfection system according to the present invention, the need for a clock or a timer for measuring how long the skin has been exposed to the disinfection fluid may advantageously be omitted. Therefore, the disinfection system may be designed more individually.

Also, as soon as the skin temperature measurement value after disinfection reaches or exceeds the prior skin temperature value (before disinfection) the disinfection process may be considered as complete.

Hence, it is possible to take environmental conditions into account during the disinfection process, e.g. room temperature, skin temperature, humidity in the environment where the disinfection system is placed, and so on. By doing so, the disinfection process may be individualized according to needs or requirements.

Information, in particular wirelessly, emitted to the user about the completed disinfection process may contribute advantageously to comply with hygienic requirements.

Also, since the disinfection system may output a signal based on the evaluation from the evaluation device for the attention of the user indicating that the disinfection process has been completed the user does not have to monitor how much time has elapsed since the disinfection fluid was dispensed. This may ease the user's work. The user himself (e. g., the doctor or the nurse) does not have to make sure that the patient's arm or skin has been properly disinfected. Rather, the patient may do so by simply waiting until the disinfection system emits a "disinfection completed" signal or the like. Hence, the medical staff may save time this way.

All the advantages achievable with the system according to the present invention and/or its devices can be in certain embodiments achieved undiminished by the method according to the present invention and vice versa.

In the following, the present invention is described based on preferred embodiments thereof with reference to accompanying drawings. However, the present invention is not to be limited to these embodiments. In the figures, in which same reference numerals denote identical or similar elements, values and so on, the following applies:
- **Fig. 1**: shows a first embodiment of a disinfection system according to the present invention;
- **Fig. 1a**: shows a second embodiment of a disinfection system according to the present invention; and
- **Fig. 2**: shows the results of various measurements of temperature values by the temperature measuring device in relation to pre-determined temperature values or ranges in a second and third embodiment.

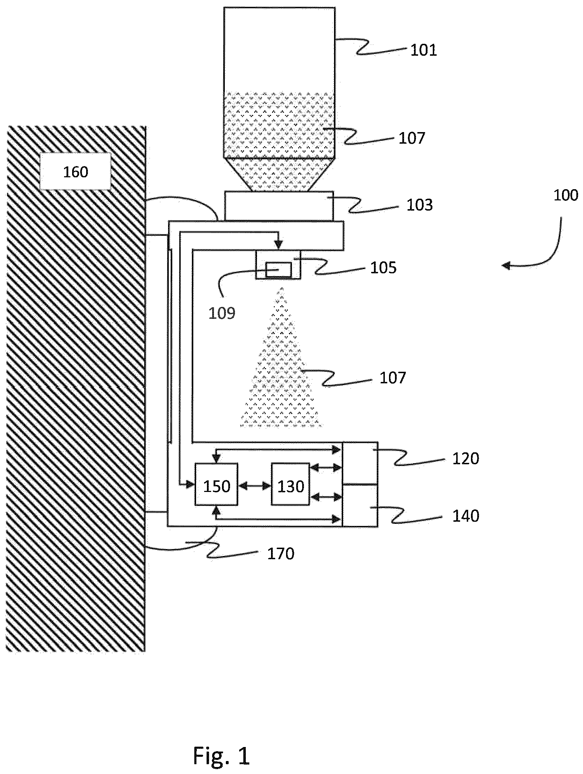

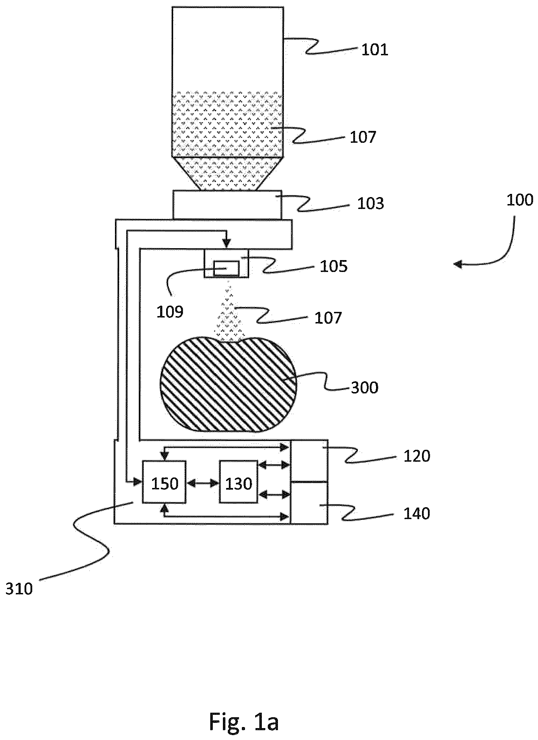

**Fig. 1** shows a first embodiment of a disinfection system 100 according to the present invention in a highly simplified side view.

On the left-hand side there is shown a wall 160 on which the disinfection system 100 is mounted by an optional wall mount 170. The wall mount 170 serves for an advantageous, comfortable and/or safe installation of the disinfection system 100 on the wall 160. It is, purely exemplarily, shaped as a clamp. Any other type of fastening device for attaching the disinfection system 100 to a wall is also comprised by the present invention.

Further, the disinfection system 100 shows a reservoir 101, optionally in the form of a bottle, which is received upside down in a receptacle 103. The optional receptacle 103 serves as a support for the reservoir 101. It may optionally be designed differently.

The reservoir 101 is fluidly connected to an applicator 105, which is designed and provided to serve for dispensing an appropriate amount of disinfection fluid 107 to the user, in particular to his or her hands. For this purpose, the applicator 105, for example, may be or comprise a nozzle serving for atomizing the disinfection fluid 107. The dispensing of the disinfection fluid 107 may be effected or triggered by an optional switch 109, which may in turn be activated manually or triggered via a sensor and an actuator (both not shown in Fig. 1). A touch-free solution may advantageously contribute to the prevention of contamination. A spray or drizzle of disinfection fluid 107 is indicated in Fig. 1 by a dotted triangle. The triangle marks the area where the body part of the user, which is to be disinfected, should be placed in order to be wetted appropriately with disinfection fluid 107.

The lower part of the disinfection system 100 reveals a temperature measuring device 120. The temperature measuring device 120 may be or comprise an infra-red measuring unit. It may be configured or suited for measuring the temperature values on or above a body part of the user, in particular his or her hands.

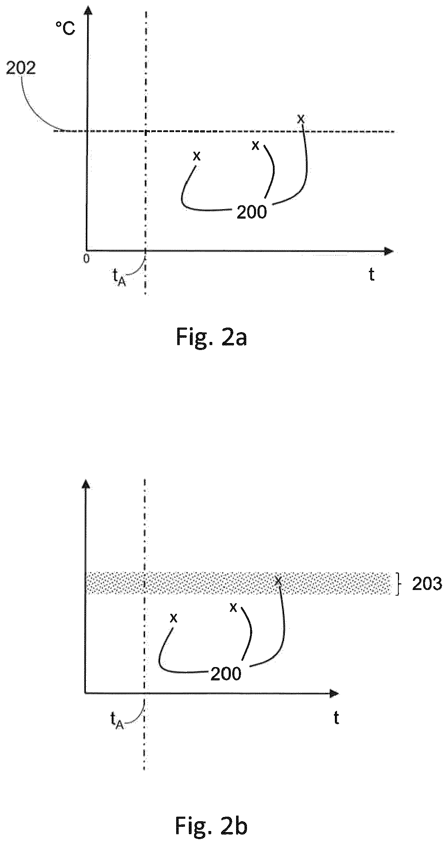

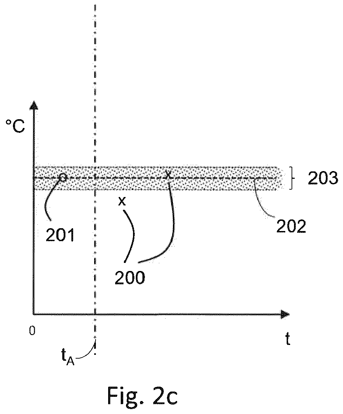

The temperature measuring device 120 is in signal communication with an evaluation device 130 which may in turn be programmed or configured to process a temperature value (see 200, 201 in Fig. 2) received from the temperature measuring device 120.

For example, the evaluation device 130 may compare the first temperature value 200 with a pre-determined or pre-set reference temperature value 201 or, alternatively, with a pre-determined temperature range 203.

In other embodiments, the evaluation device 130 may determine or set a reference temperature value 202 or a reference temperature range 203 based on a second temperature value 201, measured before the application of the disinfection fluid (see Fig. 2). The evaluation device 130 may use here the calculating steps mentioned above in the description. They are omitted here in order to avoid duplication.

The evaluation device 130 is in signal communication with an optionally provided controller 150, configured and/or programmed to control the applicator 105 and optionally the switch 109 (if provided) or, if provided, also to control a sensor and/or an actuator activating the switch 109, the evaluation device 130, the temperature measuring device 120 as well as an output device 140.

In other embodiments, it may also be considered that the dose of disinfection fluid to be dispensed is determined by the controller 150. In specific embodiments, the temperature of the environment, in which the disinfection system 100 is placed, is additionally taken into account when determining a reference temperature value or reference temperature range.

This means that the controller 150 is preferably connected in signal communication with said devices (150, 105, 109, 120, 130, 140) of the disinfection system 100. These signal communications or connections are indicated in Fig. 1 by double arrows. In specific embodiments, the controller 150 may control these devices also in a closed-loop manner.

Exemplarily, the controller 150 may trigger the output device 140 to emit a signal corresponding to the results from the evaluation device 130. For example, the signal may be an alarm signal. For example, the signal may be a signal meaning "disinfection completed", "terminated" or "done". In specific embodiments, the signal may be a signal communicating "Wait!". Optionally, the signal may be an optical signal like a light. In other embodiments, the signal may be an acoustical signal, like a tone.

It is also considered, by the present invention, that a combination of optical and acoustical signal is emitted by the output device, e.g. a red light combined with an alarming sound.

**Fig. 1a** shows a second embodiment of a disinfection system 100 according to the present invention in a highly simplified side view.

In the embodiment of Fig. 1a the disinfection system 100 is provided as a mobile device, i.e. movable or transportable and not fixed to a wall.

All other statements concerning the features of the disinfection system 100 may also apply to Fig. 1a.

With the disinfection system 100 shown in Fig 1a it is possible to disinfect parts of a patient's body without moving the patient to the disinfection system 100, as the disinfection system 100 can be moved to the patient. This may facilitate the disinfection process, for example, by disinfecting a part of the patient's body just before the next treatment step is taken, e.g., before a cannulation of a patient's arm, right at the place where this next step is taken, e.g. on a patient support, at bedside or the like.

The disinfection system 100 may comprise a stand 310 in order to place the disinfection system 100 above a part of the patient's body. The stand 310 is exemplarily provided as a C-shaped stand (open to the side) in Fig. 1a. The invention nevertheless may also comprise other types of stand, e.g. legs or the like. The stand, if provided, may be U-shaped (open to the bottom) or have any other shape. The embodiment shown is not intended to be limiting, and it is just one example of a moveable or transportable disinfection system 100 according to the present invention that may be moved to where it may be useful.

**Fig. 2a to 2c** show various temperature values resulting from various measurements by the temperature measuring device 120 in relation to pre-determined or pre-set reference temperature values 202 or reference temperature ranges 203, respectively.

Due to the wetting by an alcoholic disinfection fluid and its subsequent evaporation, the temperature of a wetted skin portion decreases measurably. The evaporation is accelerated by two factors: The warm surface (approx. 32°C) and the easily volatile alcoholic fluid. The evaporation time may depend on the room temperature or the ambient temperature.

The diagram in **Fig. 2a** shows temperature [°C] over time t and represents, in a highly simplified manner, a first disinfection process for which the disinfection system according to the present invention may be configured.

The (first) temperature values 200, indicated with an "x" in Fig. 2a, may exemplarily have been measured by the temperature measuring device 120 of an embodiment which may correspond to the embodiment in Fig. 1.

A pre-determined reference temperature value 202 is indicated by a dotted line.

The pre-determined reference temperature value 202 may have been established prior to the disinfection based on several factors, for example, the individual body temperature of the user and/or the temperature of the environment, i.e. where the disinfection system is positioned. In other embodiments, the reference temperature value 202 is established based on experience. In any case, the reference temperature value 202 would preferably be set to ensure a quick but nevertheless effective and safe disinfection. A reference temperature value 202 may exemplarily be stored in the disinfection system 100, e.g. in the evaluation device 130 or in the controller 150. A reference temperature value 202 may lie around the skin temperature of a healthy person (approx. between 28°C and 35°C).

At a time point t_{A} in Fig. 2a, 2b and 2c, disinfection fluid 107 is applied or dispensed to the user. Usually, the disinfection fluid 107 cools down the skin being disinfected.

Following the time point t_{A} the temperature on or above the hands of the user is measured several times by the temperature measurement device 120 resulting in subsequent temperature values 200 (each of which also being denoted as first temperature values herein). In the embodiment of Fig. 2a, the criterion for a successful disinfection is whether, and if so, when, one of the first temperature values 200 reaches or exceeds the pre-set (pre-determined) reference temperature value 202.

The measurements by the temperature measurement device 120 may take place at equal intervals but do not have to. They may take place at certain pre-determined time points.

As long as all first temperature values 200 are lower than the pre-determined reference temperature value 202, an alarm signal may be triggered or emitted via the output device 140 in order to indicate to the user that the disinfection process has not been completed and, thus, is not yet effective.

As soon as a first temperature value 200 reaches or exceeds (as in Fig. 2c) the pre-determined reference temperature value 202, the user may be informed via the output device 140 that the disinfection process has been completed and is effective now.

Once a signal indicating the completion of the disinfection process is emitted by the output device 140, the controller 150 may prepare the disinfection system 100, particularly the applicator 105, for the next user. For example, the first temperature values 200 may be reset to a default. Also, the reference temperature 202 may be determined based on meanwhile changed conditions.

The diagram in **Fig. 2b** shows temperature [°C] over time t and represents, in a highly simplified manner, a second disinfection process for which the disinfection system according to the present invention may be configured.

A pre-determined reference temperature range 203 is indicated by a dotted area.

All the features pointed out above for establishing the reference temperature value 202 also apply without restriction to the reference temperature range 203.

However, the criterion for a successful disinfection is, in Fig. 2b, whether one of the first temperature values 200 measured by the temperature measuring device 120 after t_{A} is found to be within the pre-set (pre-determined) reference temperature range 203.

The processing of the first temperature values 200 may be analogue or comparable to their processing in Fig. 2a.

The diagram in **Fig. 2c** shows temperature [°C] over time t and represents, in a highly simplified manner, a third and fourth disinfection process, for which the disinfection system according to the present invention may be configured.

The (first) temperature values 200, again indicated with an "x" as in Fig. 2a and Fig. 2b, as well as the (second) temperature value 201, indicated with an "o" in Fig. 2c, may exemplarily also be measured by the temperature measuring device 120 of an embodiment which may correspond to the embodiment in Fig. 1.

However, the disinfection process, particularly the determination of the reference temperature value 202 or the reference temperature range 203, respectively, is different.

During the disinfection process in Fig. 2c, a second temperature value 201 is measured before the time point t_{A}. At this time disinfection fluid 107 is applied or dispensed to the user.

This second temperature value 201 is determined or set, e.g. either by the evaluation device 130 or by the controller 150, as a reference temperature value 202.

Alternatively, or additionally, a reference temperature range 203 may be calculated on the basis of the second temperature value 201 analogue or comparable to the processes described above.

In the third disinfection process, the criterion for a successful disinfection is whether one of the first temperature values 200 measured by the temperature measuring device 120 after t_{A} reaches or exceeds the reference temperature value 202, i.e. the second temperature value 201.

In the fourth disinfection process, the criterion for a successful disinfection is whether the first temperature value 200 measured by the temperature measuring device 120 after t_{A} lies within the determined or set reference temperature range 203 calculated based on the second temperature value 201, e.g. by one or more of the mathematical methods described herein.

All the features of the diagrams in Fig. 2a and 2b regarding the measurement(s) of the first temperature values 200 and the method to check whether these first temperature values 200 fulfil at least one pre-determined criterion also apply to the process in the diagram in Fig. 2c.

The same applies to the information for the user, i.e. the output of a signal.

### Reference numerals

- 100: disinfection system
- 101: reservoir
- 103: receptacle or support for receiving the reservoir
- 105: applicator
- 107: disinfection fluid
- 109: switch
- 120: temperature measuring device; infra-red measuring unit
- 130: evaluation device
- 140: output device
- 150: controller
- 160: wall
- 170: wall mount

- 200: (first) temperature value
- 201: (second) temperature value
- 202: (pre-)determined or (pre-)set reference temperature value
- 203: (pre-)determined or (pre-)set reference temperature range

- 300: patient's arm
- 310: stand

- t_{A}: time point of application

## Claims

1. A disinfection system (100) for use by, e. g., a user, when disinfecting a part of the skin of a user or a patient, comprising
- a reservoir (101) for a disinfection fluid (107) and/or a receptacle (103) for a reservoir (101) for a disinfection fluid (107);
- an applicator (105) for dispensing disinfection fluid (107) from the reservoir (101) to the user upon request;
- a temperature measuring device (120) for measuring temperature values (200, 201) on or above a body part of the user, particularly of a skin section;
- an evaluation device (130) for the evaluating of temperature values (200, 201) measured by the temperature measuring device (120);
- an output device (140) configured to emit a signal to a medical device and/or for the attention of the user, wherein the signal is based on an evaluation carried out by the evaluation device (130) of at least one first temperature value (200) measured by the temperature measuring device (120).

2. The disinfection system (100) according to claim 1,
wherein the evaluation device (130) is programmed to compare the first temperature value (200) with a pre-determined reference temperature value (202) or with a pre-determined reference temperature range (203).

3. The disinfection system (100) according to any one of the preceding claims, wherein the temperature measuring device (120) is furthermore programmed to, preferably in an automatic manner, measure a second temperature value (201) on or above a body part of the user, wherein the first temperature value (200) is measured after the disinfection fluid (107) has been dispensed to the user from the applicator (105), and wherein the second temperature value (201) is measured before the disinfection fluid (107) is dispensed to the user from the applicator (105), and wherein the evaluating device (130) is programmed to use the second temperature value (201) as the pre-determined reference temperature (202) and/or to determine or set the pre-determined reference temperature range (203).

4. The disinfection system (100) according to any one of the preceding claims, wherein the signal emitted from the output device (140) is an alarm signal, which is given when the evaluation via the evaluation device (130) indicates that the first temperature value (200) measured by the temperature measuring device (120) does not meet at least one pre-determined criterion.

5. The disinfection system (100) according to any one of the preceding claims, wherein the signal emitted from the output device (140) is a signal indicating that the disinfection process has been completed, which is given when the evaluation via the evaluation device (130) indicates that the first temperature value (200) measured by the temperature measuring device (120) does meet at least one pre-determined criterion.

6. The disinfection system (100) according to any one of the preceding claims, wherein the temperature measuring device (120) comprises an infra-red measuring unit.

7. The disinfection system (100) according to any one of the preceding claims, wherein the disinfection fluid (107) is a disinfection solution.

8. The disinfection system (100) according to any one of the preceding claims, wherein the reservoir (101) is or comprises a bottle, or is in the form of a bottle.

9. The disinfection system (100) according to any one of the preceding claims, wherein the receptacle (103) is or comprises a reservoir (101) for or with a disinfection fluid (107).

10. The disinfection system (100) according to any one of the preceding claims, wherein the reservoir (101) comprises a disinfection fluid (107).

11. The disinfection system (100) according to any one of the preceding claims, comprising a controller (150), configured to control the applicator (105), the evaluation device (130), the temperature measuring device (120) as well as the output device (140).

12. The disinfection system (100) according to any of the preceding claims, comprising a wall mount (170).

13. A method for disinfecting a part of the body of a patient or a user, the method comprising the steps:
- providing a reservoir (101) comprising a disinfection fluid (107);
- activating an applicator (105) for dispensing disinfection fluid (107) from the reservoir (101) to a skin section upon request;
- measuring temperature values (200, 201) on or above the skin section after the disinfection fluid was dispensed using a temperature measuring device (120);
- evaluating based on the at least one first temperature value (200) measured by the temperature measuring device (120) based on a pre-determined or pre-set reference temperature value or with a pre-determined or pre-set reference temperature range.

14. The method according to claim 13, wherein the evaluating step is or encompasses comparing the measured temperature value (200) with said pre-determined or pre-set reference temperature value or range.

15. The method according to claim 13 or 14, wherein for carrying out the method a disinfection system (100) according to any one of the claims 1 to 12 is used.
